# EUROPEAN PATENT APPLICATION

(11) **EP 0 521 731 A1**
(43) Date of publication of application: **07.01.1993**
(21) Application number: 92306173.3
(22) Date of filing: 03.07.1992
(51) Int. Cl.: A61M 5/32

(54) **A syringe needle destructor**

(30) Priority: 05.07.1991 CN 91217107
(71) Applicant: Hsieh, Ch'ing-Lung, Shin Juang City, Taipei County (TW)
(72) Inventor: Hsieh, Ch'ing-Lung, Shin Juang City, Taipei County (TW)
(74) Representative: King, James Bertram

(57) **Abstract**

A syringe needle destructor has a platform (42) movable towards a housing opening (61) against spring pressure (62,63). The platform has a triangular opening (423) and a sliding shutter (41) with a reversed sense triangular opening (411). The openings (411, 422) overlap and when closed define a needle receiving opening at the apexes. A needle of a spent hyperdermic syringe is pushed through said aperture to contact and bridge electrodes (55,56) such that current flow fuses the needle. A third electrode (54) augments the destruction. The shutter (41) opens to receive a wider diameter body part of the syringe and the downward movement of platform (42) ensures the needle is melted-off as close as possible to the syringe cap. The cap is fused and fluid sealed-off in this process. The shutter prevents sparks and fragments from exiting the housing.

## Description

This invention relates to the field of medical health equipment and is specifically concerned with a means for destruction of syringe needles quickly which also prevents any residual injection fluid in the needle or cap from escaping.

The syringe needle is a daily necessity in medical health establishments which use them in quantity. Since the needle tip is very sharp and some residual injection fluid may stay in the needle cap, if directly discarded the used syringe may cause injury and spread disease. As a rule, syringes must be disposed of safely not just discarded.

A pocket syringe needle destructor in Taiwan Patent No. 7,228,127 is known. The said syringe needle destructor, as shown in Figures 1 and 2, consists of a housing 21, a needle collector 22 in the housing 21, and two electrodes 14, 15 which are indented but not in contact. A needle supporting frame device above the housing 21 consists of a needle inserting hole 211 and a horse-shoe shaped supporting frame 213 of which the width is the same as that of a needle sliding track 212. The needle inserting hole 211 is a round hole above the two electrodes 14, 15, and the horse-shoe shaped supporting frame 213 is disposed inside the needle inserting hole 211 and at the needle sliding track 212 but slightly lower, with the same width as that of the needle sliding track 212, slightly smaller than the needle shoulder 311 but slightly larger than the other part of needle. The operating principle thereof is thus: when a needle 31 is to be destroyed firstly the needle 31 is inserted in the needle inserting hole 211 so as to contact the two electrodes 14, 15 to form a loop wherefrom the high temperature generated from the current melts down and distorts the needle 321 instantaneously so that the syringe 3 moves down and the needle supporting shoulder 311 contacts and stops at the supporting frame 213, then the barrel 32 with needle 31 slides toward one side of the needle sliding track 212, and during sliding, the needle 31 destroyed, distorted and caught below the needle sliding track 212 disengages from the barrel 32 and falls down onto the metal plate 221 of needle collector 22 in the housing. The operating processes of destroying, disengaging and collecting the needle 31 are convenient and quick but the following problems remain:
1. When proceeding with the destruction of needle 31, sparks and flashes emit from the needle inserting hole 211 and the needle sliding track 212 and can distress the user.
2. If the head ring of the needle cap is extremely small or there is no needle cap at all, it is inconvenient to use such equipment.
3. The waste needle head in the needle collector has the needle cap wherein some residual injection fluid or blood may exist. If not quickly eliminated from the needle head, a bad odour may be generated in the needle destructor.
4. After the destroyed needle with plastic needle cap falls down into the needle collector 22, the red-hot metal parts of the needle cap of a following needle under destruction will fall down onto the previously destroyed plastic needle caps in the needle collector 22 leading to burning of these plastic needle caps and emission of noxious fumes.
5. If the barrel and needle of the syringe are integrally moulded or thread-engaged with each other, the needle cannot disengage from the barrel after destruction, and the melted needles will form a mass of hot metal pieces between the two electrodes 14, 15 and have to be taken out together with their barrels from the syringe needle destructor, and when discarding these parts there is a risk of burning or igniting other materials.

One of the objects of this invention is to provide a syringe needle destructor which is suitable for various needles having different shapes and specifications which is characterised by quick destruction, much less emission of sparks and flashes and avoiding burning or ignition of waste on disposal and without risk of pollution from residual injection fluid and smells.

According to this invention there is provided a destructor for syringe needles comprising a housing, a multi-electrode needle destroying device and a needle supporting frame, characterised in that the needle supporting frame is resiliently mounted and disposed above the housing and above the destroying device, said frame comprising a sliding face plate with a needle insertion hole and a resilient frame body, the housing having an opening containing a plurality of electrode members forming a multi-electrode needle destroying device, two catch members being provided each side of the opening to engage and retain the supporting frame, the opposite inner edges of said catch members being provided with two spring retainers to support two mounting spring means of the supporting frame respectively, the multi-electrode needle destroying device comprising at least two electrode members located in the housing, and connected across a power source.

In an embodiment two electrode members may comprise three-piece electrodes in the shape of flat plates or two-piece multi-layer electrodes of various geometric shapes but divided into two electrode sets so as to form two circuits.

The operating principle of the present invention is thus: the needle to be destroyed is inserted through the insertion hole of the resilient support frame. Since the support frame has a light elastic tension, it may be pressed down when subject to force and will restore to its original position when the force is removed so that the probability of emitting flashes and sparks, generated when the destruction of a needle proceeds, is reduced. The insertion hole allows destruction of a needle up to the shortest extent (about 2mm from the needle cap); the two sets of electrodes comprise two or three electrode members and the destroyed needle can form two current paths: the inserted needle and one set of electrodes form a first path which generates a high temperature quickly to destroy the needle when the heavy current passes through it so the needle under the action of the support frame moves down to be continuously destroyed until the support frame is pressed down to the limit, namely, somewhere about 2mm from the needle cap, and then lifts the syringe; the high temperature distorts and destroys the needle which is reduced to metal chips or globules which are then connected to another set of electrodes to form a second path which quickly generates a high temperature to melt the waste needle which falls into the needle collector in the housing, the short residual needle head part below the needle cap is sealed at the tubular opening under the high temperature melting, thus any injection fluid and blood residually left in the needle cap will be trapped. So far as the syringe with needle is concerned, the needle attached to the needle cap after destruction is only 2mm long, so the said syringe taken out from the housing can quickly reduce in temperature by radiant heat and will not ignite waste in a disposal bin or burn operatives.

According to this invention there is also provided a syringe needle destructor characterised by a platform movable towards a housing having an opening and against spring pressure, the platform having a triangular opening and a sliding shutter with a reversed sense triangular opening, the said openings overlapping and when closed defining a syringe needle receiving opening at the apexes thereof, a needle of a spent hyperdermic syringe being pushed through said aperture to contact and bridge electrodes forming a needle destroying device such that current flow fuses and destroys the needle, preferably a third electrode is provided to augment the destruction, the shutter opening to receive a wider diameter body part of the syringe and the downward movement of platform ensuring that the needle is melted-off as close as possible to the syringe cap.

This invention can be designed to have a needle insertion hole with variable diameter and shape so that the present invention may be suitable for various needles with different shapes and specifications.

This invention is further described and illustrated with reference to an embodiment shown in the drawings as an example. In the drawings:

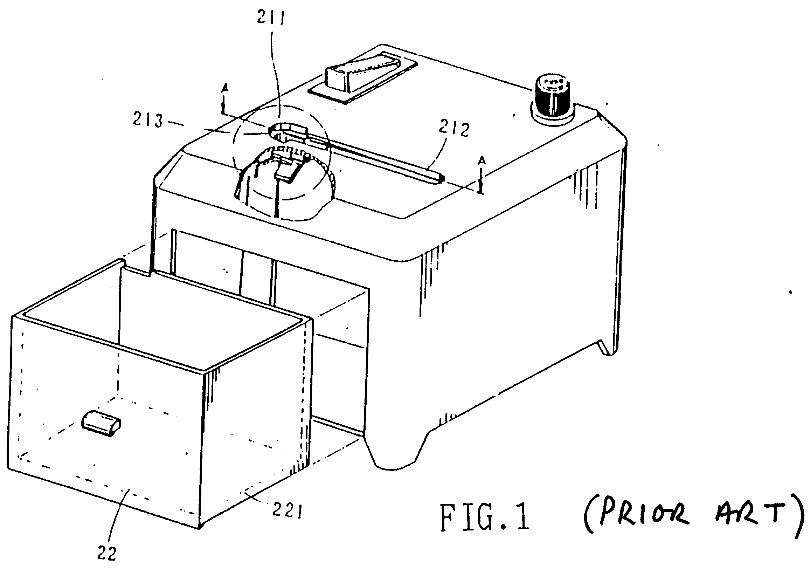

Figure 1 shows a view of a prior-art pocket syringe needle destructor,

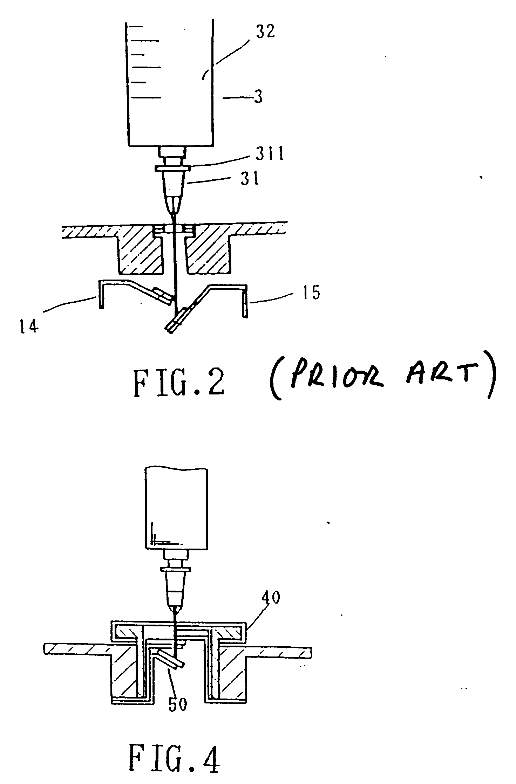

Figure 2 shows a schematic view of the structure of the pocket syringe needle destructor of Figure 1,
Figure 3 shows a view of an example of the present invention,
Figure 4 shows a schematic view of the structure of the example of Figure 3,
Figure 5 shows an exploded view of the support frame of a multi-electrode needle destroying device and electrode members of the example of the present invention,
Figure 6 shows a schematic view of the insertion hole in an open state on the support frame of the example of the present invention,
Figure 7 shows a schematic view of the insertion hole in a closed state on the support frame of the example of the present invention,
Figure 8 is a perspective view of the housing of a multi-electrode type needle destroying device of the example of the present invention,
Figure 9 is an exploded view of the support frame of a multi-electrode type needle destroying device and electrode member of a second example of the present invention, and

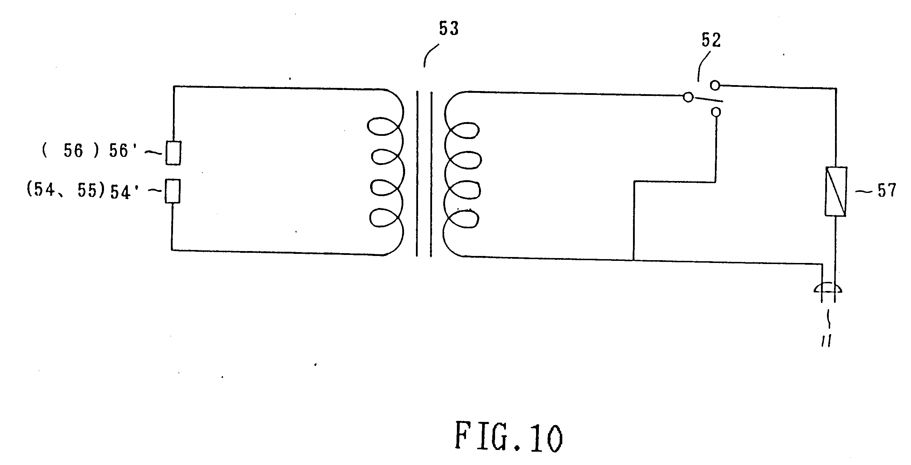

Figure 10 is an electric wiring diagram of the device of the present invention.

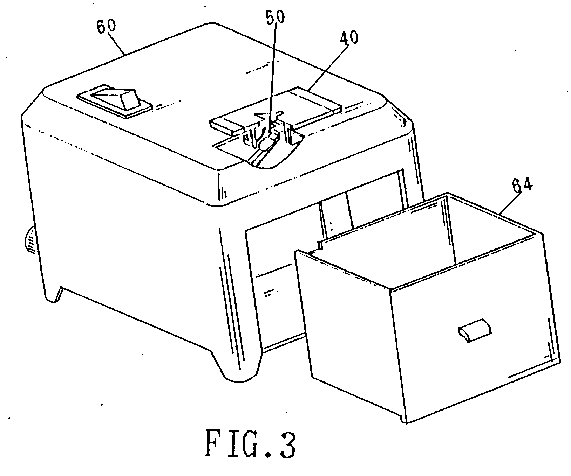

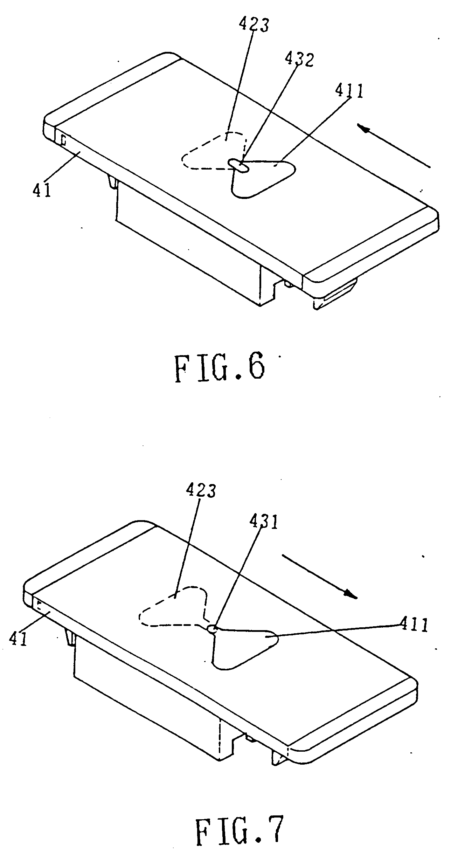

As shown in Figures 3 to 7, a first example of the present invention consists of a resiliently mounted elastic support frame 40, a multi-electrode type needle destroying device 50 and a housing 60. The elastic support frame 40 is installed above the housing 60, the destroying device 50 is installed in the housing 60 beneath the elastic support frame 40, and the elastic support frame 40 consists of a sliding face plate 41 and an elastic frame body 42 coupled with each other. The sliding face plate 41 is provided with an opening 411 of triangular shape. The front and rear side edges of sliding face plate 41 are respectively folded down to form two corresponding sliding grooves 412, 413, two corresponding notches 412a, 413a are provided in positions near the ends of said two sliding grooves 412, 413. The two ends of a retainer (damper) 414 pass through and engage between the two notches 412a, 413a. The side edge of sliding face plate 41 on the side corresponding to the damper 414 is suitably folded down to form a stop block 415. The corresponding inserting rods 416a, 416b, 416a′, 416b′ of two decorative strips 416, 416′ can just engage the two ends 412b, 413b, 412c, 413c of two corresponding sliding grooves 412, 413 on the outer side of damper 414 (or stop block 415). The elastic frame body 42 consists of a frame face 421 with a triangular opening 423 at the centre and four-piece linked (or separable) support wall 424 extended down from the four sides below the frame face 421, one corresponding catch 425, 426 is provided on the left and right outer sides of support wall 424, a leaf spring 43 is provided between the outer side of catch 426 and the stop block 415 of sliding face plate 41. A face plate 422 is provided to the frame face 421 which partially and correspondingly overlaps the triangular opening 411 on the sliding face plate 41. When the sliding face plate 41 and the face plate 422 of elastic frame body 42 displace toward the damper 414 and stop block 415, the correspondingly overlapped part of the opening 411 on the sliding face plate 41 and the opening 423 on the face plate 422 of elastic frame body 42 will be able to elastically and retractably form an insertion hole 431 of the size of the needle hole or expand up to an insertion hole 432 with a diameter of about 0.5cm, so needles of various shapes or different diameters can be inserted through these two holes 431, 432 to satisfy and meet the demand of various needles. Most of the inner edge of the opening 411 on the sliding face plate 41 does not overlap the opening 423 on the face plate 422 of the elastic frame body 42 but contacts the surface of face plate 422 of elastic frame body 42 to form a guiding ring capable of quickly guiding the needle for insertion in the insertion hole 431.

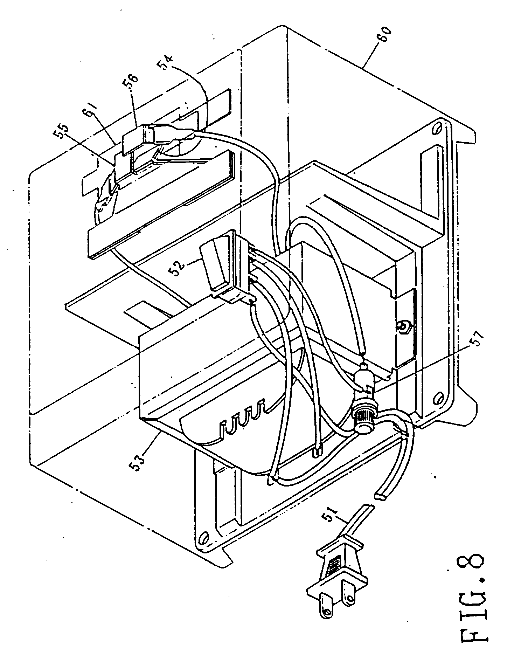

As shown in Figures 3 to 8, the multi-electrode needle destroying device 50 consists of a power line wire 51, a switch 52, a transformer 43, three electrode members 54, 55, 56 and a fuse 57 which are connected to a mains power supply source 110V or 220V. The electrode members 54, 55, 56 consist of outer layers 541, 551, 561 of platinum with a high melting point and inner layers 542, 552, 562 of copper respectively. The electrode members 54, 55 are connected to the same electrode of the power source, but the electrode member 56 is connected to the other electrode of the power source, and a spacing of about 0.5-0.7 cm is maintained between the electrode members 54, 56. The lowest electrode member 54 and the highest electrode member 56 are correspondingly disposed. The corresponding side of electrode member 54 is inclined down at a suitable angle (about 30° - 60°). Thus the two electrode members 54, 56, transformer 53, switch 52, power source wire 51 and the needle to be destroyed form a path to melt down the needle of the syringe. The electrode member 55 is disposed considerably nearer but not in contact with the highest electrode member 56. Thus the electrode member 56, transformer 53, switch 52, power source wire 51 and the needle to be destroyed form another path in order to melt down the destroyed metal chips of needles in a mass nearer the lower edge of the needle cap.

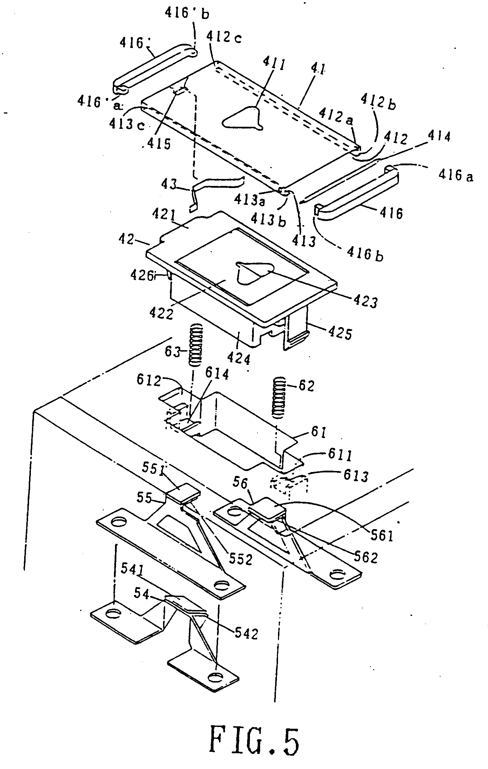

As shown in Figure 5, the three electrode members 54, 55, 56 of multi-electrode type needle destroying device 50 can be screwed into a cross opening 61 on the housing 60. A snap hole 611, 612 is provided respectively to the two sides of the cross opening 61, and a spring groove 613, 614 is provided respectively to the opposite inner edges of snap hole 611, 612 to contain a spring 62, 63 which can elastically support the support frame 40. When support the frame 40, subject to pressure, moves down to fully compress the spring 62, 63 on the lower edge of two sides of elastic frame body 42, the opening 423 on the face plate 422 of frame body 42 is very close to the highest electrode member 56 in the housing 60, so that the needle can be fully melted down (about 2mm close to the lower edge of the needle cap).

In use and in order to destroy a syringe needle, first the needle slides along the guiding ring and toward the insertion hole 431 and instantaneously enters the insertion hole 431 so as to contact the lowest electrode member 54. Since the side of electrode member 54 corresponding to the electrode member 56 is inclined down to an angle, the needle slides toward electrode member 56 and a circuit path is formed by the needle and two electrode members 54, 56. When a large current passes through this loop a high temperature is quickly generated to melt down the needle. Under the action of the support frame 40, the needle moves down on the one hand and is melted down on the other hand until the elastic support frame 40 is pressed down to the lowest position, and the needle is melted down to about 2mm from the needle cap. The user then lifts up the syringe to allow the distorted waste needle melted down to metal chips to contact the electrode members 55, 56, so that the remains of the needle, the two electrode members 55, 56 and the electronic components of power source wire 51, switch 52 and transformer 53 form the second current path to quickly generate a high temperature to melt down the remains of the needle. The distorted needle falls down into the needle collector 64. Since the tubular opening of the shortest residual needle is sealed by the high temperature, any residual injection fluid and blood in the needle cap will not flow. Finally, the residual needle adhered to the needle cap will be only 2mm long and can be removed from the insertion hole 431 (or 432). Since the volume of the said residual needle is very small and the temperature therefore will be quickly radiated and lowered, it will not ignite other material in the rubbish bin or burn the operatives. Because the insertion hole 431 is formed by overlapped parts of both openings 423, 411, and the face plate 41 corresponds to the frame face 421 for sliding, needles of various diameters and shapes can be inserted through the insertion hole 431, and then under the action of spring 43, the clearance between the insertion hole 431 and the needle is made extremely small so that the emission of sparks or light can be considerably decreased.

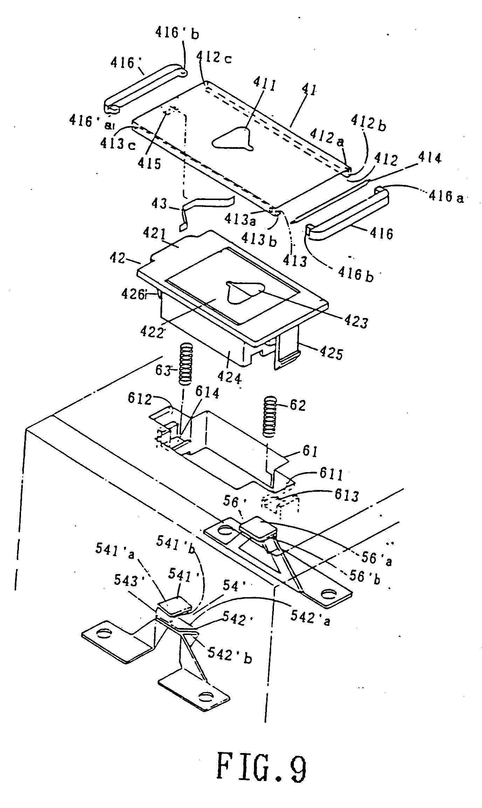

Figure 9 shows a second example of the present invention which is the same as the above said first example except for the structure of the electrode members of the multi-electrode needle destroying device. Example 2 has two electrode members: the upper electrode member 56′ remains as a flat plate type electrode member but the lower electrode member is a two-layer electrode member 54′ which consists of an upper-layer electrode member 541′, a lower-layer electrode member 542′ and a base 543′; both the upper-layer electrode member and the lower-layer electrode member are on the same one side of the base but do not extend in parallel, so a suitable contained angle is formed therebetween (about 30° - 60°); the upper-layer electrode member 541′ is narrower than the lower-layer electrode member 542′, the upper-layer and lower-layer electrode members 541′, 542′ and the electrode member 56′ consist of an outer metal layer 541′a, 542′a, 56′a and an inner copper layer 541′b, 542′b, 56′b respectively. The electrode member 56′ is connected to one electrode of a power source but the two-layer electrode member 54′ is connected to another electrode of a power source. The two-layer electrode member 541′ is parallel with the upper electrode member 56′ and close to the latter but not in contact with the latter, and the corresponding sides of both the lower-layer electrode member 542′ and the upper electrode member 56′ incline down at a suitable angle (30° - 60°). The function and usage of the two-layer electrode member 54′ are the same as those of the electrode members 54, 55 in the first example.

## Claims

1. A syringe needle destructor characterised by a platform (42) movable towards a housing having an opening (61) and against spring pressure (62,63), the platform having a triangular opening (423) and a sliding shutter (41) with a reversed sense triangular opening (411), the said openings (411, 422) overlapping and when closed defining a syringe needle receiving opening at the apexes thereof, a needle of a spent hyperdermic syringe being pushed through said aperture to contact and bridge electrodes (55,56) forming a needle destroying device such that current flow fuses and destroys the needle, preferably a third electrode (54) is provided to augment the destruction, the shutter (41) opening to receive a wider diameter body part of the syringe and the downward movement of platform (42) ensuring that the needle is melted-off as close as possible to the syringe cap.

2. A destructor for syringe needles comprising a housing (60), a multi-electrode needle destroying device (50) and a needle supporting frame (40), characterised in that the needle supporting frame (40) is resiliently mounted and disposed above the housing (60) and above the destroying device (50), said frame comprising a sliding face plate (41) with a needle insertion hole and a resilient frame body (42), the housing (60) having an opening (61) containing a plurality of electrode members forming a multi-electrode needle destroying device (50), two catch members (611, 612) being provided each side of the opening (61) to engage and retain the supporting frame (40), the opposite inner edges of said catch members (611, 612) being provided with two spring retainers (613, 614) to support two mounting spring means (62, 63) of the supporting frame (40) respectively, the multi-electrode needle destroying device (50) comprising at least two electrode members located in the housing (60), and connected across a power source.

3. A destructor as claimed in Claim 2, characterised in that the electrode members of the needle destroying device (50) comprise three electrodes (54, 55, 56) which are in the shape of flat plates connected to a power source and disposed a defined distance from each other in the housing (60) one pair of electrodes (54, 56) being positioned relatively and mutually obliquely up and down, and another pair of electrodes (55,56) being closer to each other but separated.

4. A destructor as claimed in Claim 2, characterised in that the electrode members of the multi-electrode type needle destroying device (50) comprise two electrode members (54′,56′) connected to a power source, one electrode member (54′) being a multi-layer electrode member, of which the lower-layer electrode (542′) of said multi-layer electrode member (54′) and an electrode member (56′) are correspondingly disposed a certain distance from each other and relatively and mutually obliquely up and down, the upper-layer electrode member (541′) of said multi-layer electrode member (54′) and the electrode member (56′) being parallel and considerably closer to each other but not in contact.

5. A destructor as claimed in Claim 2, characterised in that the electrode member of a multi-electrode needle destroying device comprises three electrode members of different geometric shapes positioned in the housing and connected to a power source, one pair of electrode members being positioned relatively and mutually obliquely up and down and a predetermined distance apart and another pair of electrode members being positioned considerably closer to each other but not in contact.

6. A destructor as claimed in Claim 2 or any preceding claim, characterised in that an opening (411) is provided in a sliding face plate (41) of the support frame (40), the front and rear sides of the sliding face plate being respectively folded down to form two corresponding grooves (412, 413), two corresponding notches (412a, 413a) being respectively provided at the ends of the two sliding grooves; the two ends of a damper (414) respectively engaging said two notches, the side edge of the sliding face plate on the side adjacent the damper being suitably folded to form a stop (415); tabs (416a, 416b, 416a′, 416b′) of two decorative strips (416, 416′) engaging the two end openings (412b, 413b, 412c, 413c) of two slide grooves on the outer side of the damper; and an opening (423) being provided in the face plate (422) on the frame face of the elastic frame body (42) of the support frame, the four sides below the frame face respectively extending down to form four linked or separate support walls (424) of which the outer left and right sides are respectively provided with a corresponding catch (425, 426).

7. A syringe needle destructor as claimed in Claim 2 or any preceding claim, characterised in that an opening (411) is provided in the sliding face plate (41) of the support frame (40), the front and rear sides of the sliding face plate being folded to form two slide grooves (412, 413) two corresponding notches (412a, 413a) being provided at the ends of the slide grooves; the two ends of a damper (414) engaging the two notches and extending therebetween, the side edge of a sliding face plate on the side adjacent the damper being suitably folded to form a stop (415), the corresponding tabs (416a, 416b, 416a′, 416b′) of two decorative strips (416, 416′) engaging two end openings (412b, 413b, 412c, 413c) of two slide grooves on the outer side of the damper; and an opening (423) being provided to the face plate (422) of the frame face of the frame body (42) of the supporting frame (40), the four sides below the frame face respectively extending down to form four linked or separate support walls (424) of which the outer left and right sides are respectively provided with a corresponding catch (425, 426); the electrode member of a multi-electrode needle destroying device (50) comprising three plate electrode members (54, 55, 56) connected to a power source and spaced apart a certain distance in the housing (60), one pair of electrode members (54, 56) being at correspondingly mutually oblique opposed angles and another pair of electrodes (55,56) being close together but separated from each other.

8. A destructor as claimed in Claim 2 or any preceding claim characterised in that an opening (411) is provided in the sliding face plate (41) of the support frame (40), the front and rear sides of the sliding face plate being folded to form two slide grooves (412, 413), two corresponding notches (412a, 413a) being provided at the ends of the two slide grooves; the two ends of a damper (414) engaging the two notches and extending therebetween, the side edge of a sliding face plate on the side adjacent the damper being suitably folded to form a stop (415), corresponding tabs (416a, 416b, 416a′. 416b′) of two decorative strips (416, 416′) engaging two end openings (412b, 413b, 412c, 413c) of two slide grooves on the outer side of the damper; and an opening (423) being provided to the face plate (422) of the frame face of frame body (42) of the support frame (40), the four sides below the frame face respectively extending to form four linked or separate support walls (424) of which the outer opposed sides are respectively provided with a corresponding catch (425, 426), the electrode member of a multi-electrode needle destroying device comprising two electrode members (54′, 56′) connected to a power source, one electrode member (54′) being a multi-layer electrode member, the lower-layer electrode (542′) of the multi-layer electrode member (54′) and the other electrode member (56′) being disposed a certain distance apart and being respectively mutually and obliquely up and down, and the upper-layer electrode member (541′) of the multi-layer electrode member (54′) and the electrode member (56′) being parallel and closer to each other but not in contact.

9. A destructor as claimed in Claim 2 and any preceding claim characterised in that an opening (411) is provided in the sliding face plate (41) of the support frame (40), the front and rear sides of the sliding face plate being folded down to form two slide grooves (412, 413), two corresponding notches (412a, 413a) being respectively provided at the ends of the two slide grooves, the two ends of a damper (414) engaging the two notches and extending therebetween, the side edge of sliding face plate on the side adjacent the damper being suitably folded to form a stop (415); corresponding tabs (416a, 416b, 416a′, 416b′) of two decorative strips (416, 416′) engaging two end openings (412b, 413b, 412c, 413c) of two slide grooves on the outer side of the damper; and an opening (423) being provided in the face plate (422) of the frame face of frame body (42) of the support frame (40), the four sides below the frame face respectively extending to form four linked or separate support walls (424) of which the outer opposed sides are respectively provided with a corresponding catch (425, 426), the electrode member of a multi-electrode needle destroying device comprising three electrode members of different geometric shapes located in the housing and connected to a power source, one pair of electrode members being positioned respectively mutually and obliquely up and down and spaced a certain distance from each other but not in contact.

10. A destructor as claimed in Claims 2, 6, 7, 8 and 9 and any other preceding claim, characterised in that the sliding face plate (41) of the support frame (40) is coupled to the frame body (42), the four sides below the frame face of the frame body (42) respectively extending down to form four connected or separate support walls (424) of which the outer sides are respectively provided with a corresponding catch (425, 426), a spring means (43) being disposed between the outer side of one catch (426) and a stop (415) of the sliding face plate; the opening (411) on the sliding face plate (41) and the opening (423) on the face plate of the frame body being partially overlapped to define an aperture of a size and shape to form an insertion hole (431), that part of opening (411) not overlapped by the opening (423) on the face plate forming a guide for the needle insertion hole on the surface of face plate (422).
